# EUROPEAN PATENT APPLICATION

(11) **EP 4 755 443 A1**
(43) Date of publication of application: **10.06.2026**
(21) Application number: 24218420.8
(22) Date of filing: 09.12.2024
(51) Int. Cl.: A61N 1/375

(54) **HEADER OF AN IMPLANTABLE MEDICAL DEVICE**

(71) Applicant: BIOTRONIK SE & Co. KG, 12359 Berlin (DE)
(72) Inventor: Klenner, Rolf, 14552 Michendorf (DE); Egart, Boris, 13353 Berlin (DE); Dust, Christian, 16356 Werneuchen (DE); Große, Ines, 14612 Falkensee (DE); Dettmer, Alexander, 10317 Berlin (DE); Straub, Mathias, 12347 Berlin (DE); Richter, Caroline, 13088 Berlin (DE); Klein, Moritz, 10589 Berlin (DE); Hippel, Robert, 12587 Berlin (DE); Ebert, Henning, 12437 Berlin (DE)
(74) Representative: Biotronik Corporate Services SE

(57) **Abstract**

The present invention relates to an implantable medical device (1), comprising: a header (10) comprising a header body (11) having a cavity (12), and a sealing plug (2) arranged in the cavity (12) to seal the cavity (12), wherein the sealing plug (2) is retained in the cavity (12) by an undercut (120) comprised by the cavity (12). According to the invention, the undercut (120) is formed by a plate (3) connected to the header body (11), the plate (3) comprising a through-hole (30) providing access to the cavity (12) for arranging the sealing plug (2) therein through the through-hole (30).

## Description

The present invention relates to an implantable medical device comprising a header. Such headers may e.g. be made by way of plastic injection molding and provide electrical connectivity to an implantable medical device, for instance for connecting an electrode lead to the implantable medical device via the header. The corresponding connector integrated into the header is typically connected to an electronic circuitry enclosed by a casing of the implantable medical device via an electrical feedthrough that may comprise electrically conducting pins that are embedded in or extend through an electrically insulating body and may be connected to the connector by a suitable wiring. The feedthrough may be covered by the header.

In order to fix a plug of an electrode lead that is inserted into a connector of the header, the header usually comprises a cavity for accommodating a screw by means of which the plug may be secured once inserted into the connector. This prevents the plug from being pulled out of the connector unintentionally. However, such a cavity needs to be sealed by a suitable sealing plug before implantation of the implantable medical device into the human or animal body.

In order to be able to retain the sealing plug in the cavity, the latter may comprise an undercut, i.e. a feature of the cavity where a portion of the material is removed in such a way that it leaves a recessed or indented area, creating an overhang or a geometric shape that prevents easy extraction of the sealing plug. Particularly, due to the undercut, the cavity comprises an opening providing access to the cavity, which opening has an inner diameter being smaller than the inner diameter of the cavity. Once the compressed elastic sealing member has been inserted into the cavity it can re-expand and is prevented from moving out of the cavity by the undercut. As this also applies to a tool or mold used to make the header and cavity therein, the undercut is an important consideration in the design and manufacturing processes. Particularly, undercuts may pose challenges because they may hinder the removal of cutting tools, molds, or dies from the workpiece or the formed part. Particularly in injection molding, undercuts may complicate the mold design and increase the complexity of the tooling.

In previous epoxy resin headers known in the prior art, a large undercut at the position of the sealing plug may be demolded from the mold, so that a corresponding retention of the sealing plug can be achieved. Furthermore, silicone sealing plugs are known for sealing the header's cavity for the fixing screw, wherein the remaining opening is sealed with a silicone adhesive.

However, in case the header is to be produced by means of plastic injection molding no undercut can be produced in the single-axis demolding process. Although rotary movements (thread, bayonet) are possible, they make both components (header and sealing plug) more expensive and more complicated to assemble.

Regarding the process variant utilizing said silicone sealing plug secured with a silicone adhesive, there is a risk of liquid penetrating the edge area of the applied silicone adhesive after a long time in the body, which could lead to insulation defects.

Based on the above, the problem to be solved by the present invention is to provide an implantable medical device having a header that may be manufactured in a cost-efficient way and allows a secure sealing of a cavity of the header. Particularly, it is a further objective to achieve demoldability of the header in the injection mold as well as a subsequent automated assembly. Ideally the solution shall allow to press in and retain the sealing plug in the cavity above a fixing screw. Furthermore, ideally, the sealing plug shall be prevented from being inadvertently pulled out when a torque spanner is released from the screw.

This problem is solved by an implantable medical device having the features of claim 1 and by a method having the features of claim 10. Preferred embodiments of these aspects are stated in the corresponding dependent claims and are described below.

According to claim 1, an implantable medical device is disclosed, comprising
- a header comprising a header body having a cavity, and
- a sealing plug arranged in the cavity to seal the cavity, wherein the sealing plug is retained in the cavity by an undercut comprised by the cavity,
wherein the undercut is formed by a plate connected to the header body, the plate comprising a through-hole providing access to the cavity for arranging the sealing plug therein through the through-hole.

Particularly, the cavity of the header body is accessible from an outside of the header or header body, wherein the cavity is configured to receive the sealing plug.

Thus, particularly, for a pre-assembled header made e.g. by plastic injection molding, a way of creating an undercut is proposed to secure the sealing plug, e.g., for sealing a fastening device, e.g., a fixing screw, at least partly arranged in the cavity. Since an injection molding tool cannot demold such an undercut itself, an additional component in form of the plate is utilized to achieve the securing mechanism.

Particularly, according to an embodiment, the through-hole of the plate comprises an inner diameter, wherein this inner diameter is smaller than an inner diameter of the cavity.

Particularly, the header of the implantable medical device is configured to receive and to electrical contact element a plug of an electrode lead, wherein at least one connector configured to receive and electrically contact the plug is accommodated in the header, the at least one connector being accessible from the outside via the cavity. Furthermore, the header of the implantable medical device is particularly configured to provide electrically conductive connections to an electronic circuitry accommodated in a housing of the implantable medical device, wherein the header is arranged on the housing, wherein the electrically conductive connection between the header, particularly the at least one connector, and the electronic circuitry may be established through an electrical feedthrough that hermetically seals the housing and comprises feedthrough pins embedded in or extending through an electrically insulating body, e.g., made from a ceramic such as alumina (Al₂O₃), a glass, a glass solder or a plastic material. At least one feedthrough pin, e.g., made from platinum, iridium, niobium, platinum/iridium, or titanium, may be connected to a connector (e.g. socket) integrated into the header body. In this way, e.g. a plug of an electrode lead inserted into the connector may be connected to the electronic circuitry. The header may be arranged on the housing so as to cover the electrical feedthrough. Particularly, the at least one feedthrough pin is hermetically joined with the electrically insulating body, particularly by brazing. Particularly, the electrically insulating body is hermetically joined with the housing, particularly by brazing.

According to an embodiment of the implantable medical device, the header body comprises a recess in which an opening to the cavity is arranged, wherein the plate is arranged in the recess, particularly such that an outside of the plate is flush with an adjacent outside of the header.

Further, according to an embodiment of the implantable medical device, the plate is bonded to the header body, particularly by a weld seam, particularly by ultrasonic welding or laser welding.

According to one embodiment of the implantable medical device, the plate is manufactured from the same material as the header body. Furthermore, in an embodiment of the implantable medical device, the material of the header and/or the plate is a plastic material, particularly a biocompatible plastic material. In one embodiment, material a thermoplastic material, particularly a transparent thermoplastic material. In one embodiment, the material is an injection-moldable plastic material, particularly a transparent injection-moldable plastic material, a thermoplastic polyurethane.

According to a further embodiment of the implantable medical device, the header body is an injection molded plastic header body. Likewise, the plate may be an injection molded plastic plate, preferably made of the same material as the header body.

Furthermore, in an embodiment of the implantable medical device, the header comprises a fastening device, particularly a fixing screw having a head accommodated in the cavity. Further, in an embodiment, the header comprises a connector (e.g. a socket) embedded in the header body, the connector being configured to receive a plug, particularly a plug of an electrode lead, wherein the fastening device, particularly the fixing screw, is configured to fasten the plug when the plug is inserted in the connector.

Further, according to an embodiment of the implantable medical device, the sealing plug is compressible so as to allow the sealing plug to be pressed through the through-hole of the plate into the cavity.

According to one embodiment of the implantable medical device, the sealing plug is arranged in the cavity to seal the cavity and cover the fastening device, particularly the fixing screw.

Furthermore, according to an embodiment of the implantable medical device, the sealing plug comprises a sealable passage for passing a tool therethrough for engaging with the fixing screw for fastening or unfastening the fixing screw. Particularly, the sealing may be achieved by opposing portions of the passage of the sealing plug butting against one another to seal the passage when the tool is removed. In one embodiment, the sealable passage is formed by cross slot or cross recess in the sealing plug, wherein particularly the slot or recess extends along the direction along which the tool is intended to pass through the sealing plug.

In a further embodiment of the implantable medical device, the sealing plug is formed from a elastic materials, particularly an elastic polymer, or a polysiloxane (silicone).

A further aspect of the present invention relates to an assembly comprising a compressible sealing plug and a header body comprising a cavity that accommodates a fastening device, particularly a fixing screw, the sealing plug being arranged in the cavity to seal the cavity.

In an embodiment of the assembly, the fastening device, particularly the fixing screw, is configured to fasten a plug received in a connector integrated into the header body. In one embodiment, the connector is designed as a pin receptacle configured to receive and electrically contact the plug, particularly a proximal portion of the plug, wherein particularly the proximal portion of the plug is designed as a pin.

In one embodiment, the assembly comprises a further connector accommodated in the header body, wherein the further connector is configured to receive and electrically contact the plug of the electrode lead, particularly an electrical contact on plug, particularly formed by a ring electrode, wherein the electrical contact on the plug is located distal from and being electrically insulated from the proximal portion of the plug.

Furthermore, another aspect of the present invention relates to a header, particularly for a medical device, the header comprising:
- a header body having a cavity, wherein particularly the cavity is characterized by a first inner diameter, and
- a plate having a through-hole, wherein particularly the through-hole is characterized by a second inner being particularly smaller than the first inner diameter,
wherein
the plate is connected to the header body such that through-hole provides access to the cavity, particularly for arranging a sealing plug in the cavity through the through-hole, and the plate forms an undercut of the cavity, wherein particularly the undercut is configured to retain the sealing plug arranged in the cavity to seal the cavity.

Particularly, the cavity of the header body is accessible from an outside of the header or header body, wherein the cavity is configured to receive the sealing plug.

In one embodiment, the header according to the invention comprises at least one connector arranged in the header body and being configured to receive and electrically connect a plug of an electrode lead, wherein the at least one connector is arranged adjacent the cavity. In one embodiment, the header further comprises a fastening device configured to fasten or unfasten a plug inserted or received in the at least one connector, wherein the fastening device is arranged at the at least one connector such that the fastening device is accessible from the outside of the header via the cavity, particularly such the fastening device is actuatable or can be actuated from the outside of the header.

All embodiments and features described or claimed herein with respect to the implantable medical device may also be used to further characterize the header according to the invention. A further aspect of the present invention relates to a medical device, particularly an implantable medical device, comprising a header according to the above-described aspect of the invention directed to a header.

According to yet another aspect of the present invention, a method for manufacturing a header for an implantable medical device is disclosed, particularly an implantable medical device according to the present invention, the method comprising the steps of:
- providing a header body of the header, wherein the header body comprises a cavity, and
- forming an undercut of the cavity by connecting a plate to the header body, the plate comprising a through-hole providing access to the cavity, particularly for arranging the sealing plug therein through the through-hole.

According to an embodiment of the method, providing the header body corresponds to injection molding the header body, wherein particularly the cavity of the injection molded header body does not comprise an undercut at an opening of the cavity for retaining a sealing plug as a result of said injection molding. Furthermore, in an embodiment of the method, the plate is injection molded, too.

According to an embodiment of the method, the method further comprises arranging a compressible sealing plug in the cavity through the through-hole of the plate to seal the cavity, wherein the sealing plug is retained in the cavity by the undercut that has particularly been provided by the plate after demolding of the header body.

According to an embodiment of the method, the header body further comprises a recess in which the opening to the cavity is arranged, and wherein the plate is arranged in the recess, particularly such that an outside of the plate is flush with an adjacent outside of the header, particularly outside of the header body.

According to an embodiment of the method, the plate is bonded, particularly welded, to the header body, particularly by one of: ultrasonic welding, laser welding.

Furthermore, according to an embodiment of the method, the plate is manufactured (e.g. injection molded) from the same material as the header body, wherein particularly the material is a plastic material, particularly a biocompatible plastic material. In one embodiment, material a thermoplastic material, particularly a transparent thermoplastic material. In one embodiment, the material is an injection-moldable plastic material, particularly a transparent injection-moldable plastic material, a thermoplastic polyurethane.

In the following, embodiments of the present invention as well as further features and advantages of embodiments of the present invention shall be described with reference to the Figures, wherein
- Fig. 1: shows an exploded view of a detail a header of an embodiment of an implantable medical device according to the invention, wherein a plate is used to create an undercut for retaining a sealing plug that seals a cavity that accommodates a fixing screw, and
- Fig. 2: shows a detail of a cavity and fixing screw of Fig. 1, and
- Fig. 3: shows a cross-sectional view of the cavity, undercut, sealing plug and fixing screw.

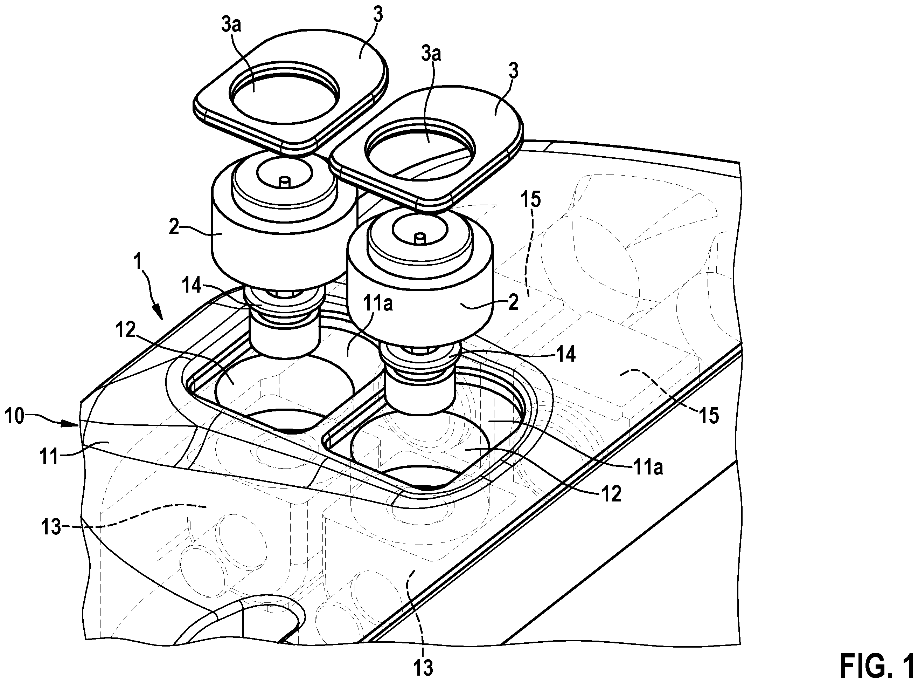

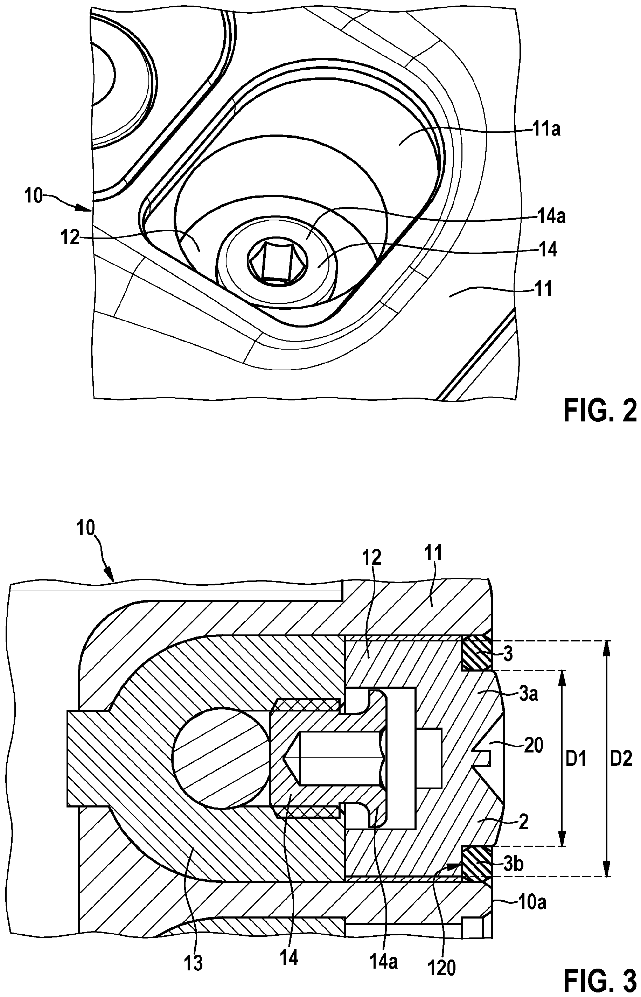

Fig. 1 shows in conjunction with Figs. 2 and 3 an embodiment of an implantable medical device 1 according to the present invention, e.g. an implantable cardiac pacemaker. The implantable medical device 1 comprises a header 10 comprising a header body 11 having a cavity 12, and a sealing plug 2 arranged in the cavity 12 to seal the cavity 12, wherein the sealing plug 2 is retained in the cavity 12 by an undercut 120 of the cavity 12. As particularly shown in Fig. 3, the undercut 120 is provided by a plate 3 connected to the header body 11. The plate 3 may be a flat plate that comprises a through-hole 3a for providing access to the cavity 12 allowing to arrange the sealing plug 2 therein through the through-hole 3a.

Particularly, the through-hole 3a of the plate 3 comprises an inner diameter D1, wherein this inner diameter D1 is smaller than an inner diameter D2 of the cavity 12. For example, through-hole 3a may have an inner diameter D1 of approx. 3 mm, and the cavity 12 may have an inner diameter D2 of approx.. 4.2 mm. Generally, the header body 11 may comprise several cavities 12 that are to be sealed by corresponding sealing plugs 2 (e.g. two such cavities 12 as shown in Fig. 1). In the following only one such cavity 12 and associated components are described in detail for the sake of brevity. However, the other cavity may be designed and sealed as will be described in detail in the following with respect said one cavity 12 as well.

Particularly, the header 10 may comprise a fastening device designed as a fixing screw 14 having a head 140 accommodated in the cavity 12 (cf. Fig. 3), wherein the header 10 may further comprise a connector 13 (e.g. in form of a socket) embedded in the header body 11, wherein the connector 13 is designed to receive a plug, e.g., of an electrode lead, so that the electrode lead can be connected via the header 10 to an electronic circuit of the implantable medical device 1. With help of the fixing screw 14 said plug may be fixed to the connector 13 when it is inserted into the connector 13. Particularly, the connector 13 may designed as a pin receptacle configured to receive and electrically contact the plug of the electrode lead, particularly a proximal portion of the plug. The plug may further comprises at least one further electrical contact, e.g., designed in form of a ring electrode, being distal from and being electrically insulated from the proximal portion, wherein the at least one further electrical contact may be electrically contacted by a further or second connector 15 accommodated in the header 10, the further or second connector 15 being particularly formed by an electrically conductive spring element, e.g., a leaf spring or a coil spring, accommodated in an electrically conductive housing, e.g., a sleeve.

Particularly, the plate 3 may be manufactured from the same material as the header body 11 itself in addition to the header body 11. Preferably, the header body 11 is an injection molded plastic header body. Likewise, the plate 3 can be an injection molded plastic plate 3. Furthermore, preferably, the plate 3 is placed on a lowered header body plateau provided by a corresponding recess 11a formed in the header body 11 (e.g. upon injection molding) and bonded to the header body 11 by e.g. ultrasonic or laser welding. Particularly, the material of the header body 11 and the plate 3 may be formed by a plastic material, particularly a biocompatible plastic material, particularly a biocompatible injection-moldable plastic material, particularly a transparent injection-moldable plastic material, particularly a thermoplastic polyurethane. Due to the recess 11a, an outside 3b of the plate 3 can be flush with an adjacent outside 10a of the header 10 or header body 11, to achieve a smooth surface of the header 10.

Furthermore, the sealing plug 2 is pressed through the constriction/through-hole 3a (inner diameter D1) of the plate 3 to seal the cavity 12 and thus has sufficient grip due to the undercut 120 to prevent it from being accidentally pulled out when a tool such as torque spanner is pulled, e.g., out of a passage 20 of the sealing plug 2.

The main advantage of the solution according to the present invention is the general ability to produce an undercut without relying on creation of the latter upon forming the actual header body. Particularly, upon injection molding of the header body the undercut cannot be realized in the basic tool of the header body. Furthermore, particularly the welding process to bond the plate providing the undercut to the header body produces an adhesive-free, secure connection between the two components, the assembly of which can be used independently of the production line and, under certain circumstances, can also be outsourced to the injection molding supplier. The additional component in form of the plate can be a simple part with no major geometric or mechanical requirements and is therefore easy to manufacture and assemble.

## Claims

1. An implantable medical device (1), comprising:
- a header (10) comprising a header body (11) having a cavity (12), and
- a sealing plug (2) arranged in the cavity (12) to seal the cavity (12), wherein the sealing plug (2) is retained in the cavity (12) by an undercut (120) comprised by the cavity (12),
**characterized in that**
the undercut (120) is formed by a plate (3) connected to the header body (11), the plate (3) comprising a through-hole (3a) providing access to the cavity (12).

2. The implantable medical device according to claim 1, wherein the header body (11) comprises a recess (11a) adjacent the cavity (12), and wherein the plate (3) is arranged in the recess (11a).

3. The implantable medical device according to claim 1 or 2, wherein the plate (3) is bonded to the header body (11), particularly by a weld seam.

4. The implantable medical device according to one of the preceding claims, wherein the plate (3) is manufactured from the same material as the header body (11), wherein particularly the material is a biocompatible plastic material, particularly a biocompatible inj ection-moldable plastic material, particularly a transparent injection-moldable plastic material, particularly a thermoplastic polyurethane.

5. The implantable medical device according to one of the preceding claims, wherein the header body (11) is an injection molded plastic header body, and/or wherein the plate (3) is an injection molded plastic plate (3).

6. The implantable medical device according to one of the preceding claims, wherein the header (10) comprises a fastening device (14), particularly a fixing screw (14) having a head (140), accommodated in the cavity (12), wherein particularly the header (10) comprises a connector (13) embedded in the header body (11), the connector (13) being configured to receive a plug of an electrode lead, wherein the fastening device (14) is configured to fasten the plug when the plug is inserted in the connector (13).

7. The implantable medical device according to one of the preceding claims, wherein the sealing plug (2) is compressible so as to allow the sealing plug (2) to be pressed through the through-hole (3a) of the plate (3) into the cavity (12).

8. The implantable medical device according to one of the preceding claims, wherein the sealing plug (2) is arranged in the cavity (12) to seal the cavity (12) and cover the fastening device (14), wherein particularly the sealing plug (2) comprises a sealable passage (20) for passing a tool therethrough for engaging with the fastening device (14) for fastening or unfastening the fixing screw.

9. A header (10) for an implantable medical device, comprising:
- a header body (11) having a cavity (12), wherein particularly the cavity (12) is **characterized by** a first inner diameter,
- a plate (3) comprising a through hole (3a), wherein particularly the though-hole (3a) is **characterized by** a second inner diameter being particularly smaller than the first inner diameter,
**characterized in that**
the plate (3) is connected to the header body (10) such that the through-hole (3a) provides access to the cavity (12), and the plate (3) forms an undercut (120) of the cavity (12), wherein particularly the undercut (120) is configured to retain a sealing plug (2) arranged in the cavity (12) to seal the cavity (12).

10. A method for manufacturing a header (10) for an implantable medical device, the method comprising the steps of:
- providing a header body (11) of the header (10), wherein the header body (11) comprises a cavity (12), and
- forming an undercut (120) of the cavity (12) by connecting a plate (3) to the header body (11), the plate (3) comprising a through-hole (3a) providing access to the cavity (12).

11. The method according to claim 10, wherein the header body (11) is provided by injection molding.

12. The method according to claim 10 or 11, wherein the method further comprises arranging a sealing plug (2) in the cavity (12) through the through-hole (3a) to seal the cavity (12), wherein the sealing plug (2) is retained in the cavity (12) by the undercut (120).

13. The method according to one of the claims 10 to 12, wherein the header body (11) further comprises a recess (11a), and wherein the plate (3) is arranged in the recess (11a).

14. The method according to one of the claims 10 to 13, wherein the plate (3) is bonded to the header body (11), particularly by one of: ultrasonic welding, laser welding.

15. The method according to one of the claims 10 to 14, wherein the plate (3) is manufactured from the same material as the header body (11), wherein particularly the material is a plastic material, particularly a biocompatible plastic material, particularly a biocompatible injection-moldable plastic material, particularly a transparent injection-moldable plastic material, particularly a thermoplastic polyurethane.
